# EUROPEAN PATENT APPLICATION

(11) **EP 1 990 423 A1**
(43) Date of publication of application: **12.11.2008**
(21) Application number: 07107960.2
(22) Date of filing: 10.05.2007
(51) Int. Cl.: C12Q 1/68, G01N 33/574

(54) **Method of determining the time to progression of non small cell lung cancer after chemotherapy based on thioredoxin expression**

(71) Applicant: Pangaea Biotech, S.A., 08028 Barcelona (ES)
(72) Inventor: Rosell Costa, Rafael, E-08916 Badalona (ES); Tarón Roca, Miguel, E-08916 Badalona (ES)
(74) Representative: ABG Patentes, S.L.

(57) **Abstract**

The present invention relates to a method for predicting the Time to Progression (TTP) of non small cell lung carcinoma (NSCLC) patients treated with chemotherapy, based on the detecting the levels of expression of thioredoxin (TRX) in a biological sample of the patient, or based on simultaneously detecting the expression levels of thioredoxin and the methylation status of the 14-3-3σ gene.

## Description

### FIELD OF THE INVENTION

The present invention relates to the field of diagnostics, in particular to a method for predicting the Time to Progression (TTP) of non small cell lung carcinoma (NSCLC) patients, based on the levels of expression of thioredoxin (TRX) as determined in a biological sample of the patient. It also relates to the use of chemotherapeutic agents selected according to the results of the previous method for the treatment of NSCLC patients.

### BACKGROUND OF THE INVENTION

Non-small-cell lung cancer (NSCLC) accounts for approximately 80% of all lung cancers, with 1.2 million new cases worldwide each year. NSCLC resulted in more than one million deaths worldwide in 2001 and is the leading cause of cancer-related mortality in both men and women (31% and 25%, respectively). The prognosis of advanced NSCLC is dismal. A recent Eastern Cooperative Oncology Group trial of 1155 patients showed no differences among the chemotherapies used: cisplatin/paclitaxel, cisplatin/gemcitabine, cisplatin/docetaxel and carboplatin/paclitaxel. The overall five-year survival of patients with NSCLC has remained at less than 15% for the past 20 years. Stage grouping of TNM subsets (T = primary tumor; N = regional lymph nodes; M = distant metastases) permits the identification of patient groups with similar prognosis and treatment options. Five-year survival is around 25% for pathologic stage IIB (T1-2N1M0, T3N0M0), 13% for stage IIIA (T3N1M0, T1-2-3N2M0), and a low 7% for stage IIIB (T4N0-1-2M0). No clinical parameters can completely account for the striking differences in survival among patients with advanced disease, with some surviving years and others only a few months.

To further improve the survival rate in NSCLC patients, their prognostic classification based on molecular alterations is crucial. Such classification will provide more accurate and useful diagnostic tools and, eventually, more effective therapeutic options.

The international patent application WO06119980, describes a method for determining whether a lung cancer is sensitive to the treatment with a combination of EGFR inhibitor and a chemotherapeutic agent which comprises determining the overexpression of phosphorylated AKT protein and/or phosphorylated MAPK protein whereby the overexpression of the phosphorylated AKT protein and/ or the phosphorylated MAPK protein is an indication that the biological sample comprising human lung cancer cells is sensitive to a combination of a epidermal growth factor receptor inhibitor and a chemotherapeutic agent. This document is concerned with predicting sensitivity to a particular chemotherapy treatment rather than in predicting TTP or survival in already treated patients.

The international patent application WO2006/097346 describes the correlation between patient survival after cisplatin-based chemotherapy and methylation levels in the 14-3-3σ gene. 14-3-3σ is a member of the 14-3-3 superfamily that is responsible for G₂ cell cycle checkpoint control in response to DNA damage in human cells. Its function has been analyzed in the human colorectal cancer cell line HCT116 (expressing 14-3-3σ and wild-type p53). Following ionizing irradiation, 14-3-3σ sequestered Cdc2/cyclin B1 complexes in the cytoplasm, thus arresting cells in G₂ and preventing them from initiating mitosis before repair to their damaged DNA. Colon carcinoma cells lacking 14-3-3σ treated with adriamycin can still initiate -but do not maintain- G2 arrest, leading to mitotic catastrophe and cell death. The expression of 14-3-3σ is reduced by p53 gene inactivation and by silencing of 14-3-3σ gene via methylation of CpG islands.

Another protein that is thought to be involved in cancer progression is thioredoxin (TRX). It is a low molecular weight redox protein that is required, in its reduced form, for the activity ofribonucleotide reductase, an enzyme essential for DNA synthesis. TRX is also a ROS scavenger and a negative regulator of apoptosis signal regulating kinase-1 (ASK-1); and exerts redox control over a number of transcription factors including NF-ₖB, human transcription factor-1C, BZLF1, the glucocorticoid receptor, and indirectly through the nuclear redox protein HAP1/Ref-1, AP-1. TRX modulates the binding of these transcription factors to DNA and thus regulates gene transcription. TRX expression is induced by a variety of forms of stress, including viral infection, mitogens, X-ray and UV irradiation, hydrogen peroxide, and postischemic reperfusion.

Recent studies have shown that TRX offers a survival as well as a growth advantage to tumours *in vivo.* Human TRX is identical to adult T-cell leukaemia-derived factor, which has growth-promoting effects on transformed cells. Consequently, TRX promotes cell proliferation and has been demonstrated to increase the growth rate and colony formation of nonmalignant and malignant cells (Powis,G. et al., Chem Biol Interact., 1998, 111-112: 23, 24).

In addition, TRX increases oxidant and drug resistance of various cells, and has been associated with resistance to drugs in certain tumours. For instance, in a gene expression profiling study, Iwao-Koizumi et al (J Clin Oncol., 2005, 23: 422) found that overexpression of TRX, together with other redox genes predict docetaxel resistance in human breast cancer. Kim et al. (Clin Cancer Res., 2005, 11: 8425), described that high TRX protein expression in breast cancer tumour is associated with resistance to docetaxel; and Arnold et al. (Cancer Res., 2004, 64: 3599) demonstrate that TRX is downstream of Smad7 in a pathway that promotes growth and suppresses cisplatin-induced apoptosis in pancreatic cancer. There are reports that transformed cells transfected with TRX and expressing high levels of the TRX protein become resistant to anticancer drugs (Rundolf et al., Antioxid Redox Signal, 2004, 6: 41). Finally, TRX, independent of the caspase apoptotic pathway, is an important determinant of resistance of cells to cell death induced by histone deacetylase inhibitors (Ungersted et al., PNAS, 2005, 102: 673).

The alleged role of TRX in different types of tumours has led to the proposal that cancer, including lung cancer, could be treated using oligonucleotides directed against TRX which are capable of modulating its expression. For instance, the US patent application US20040241717 describes a method for the treatment of cancer, including lung cancer, which consists on the administration of antisense oligonucleotides specific for TRX which are capable of modulating TRX levels in tumour cells. This document is however silent about any prognostic value of TRX expression.

Increased expression levels of TRX have been described in different lung tumours. It has been reported that almost half of human primary lung cancers overexpress TRX mRNA as compared with normal lung tissue from the same subjects (Berggren, M et al. Anticancer Res., 16: 3459-3466, 1996). Kakolyris,S. et al. (Clin. Cancer Res., 2001, 7:3087-3091) describe a strong association between absence of TRX expression and regional lymph node negativity in NSCLC samples. Soini, Y. et al. (Clin. Cancer Res., 2001, 7:1750-1757) describe an increased expression of TRX protein and mRNA in tumour samples isolated from NSCLC patients in comparison with non-neoplastic lung and Kim,H.J. et al. (Cell Biol. Toxicol., 2003, 9:285-298) describe an increased expression of TRX in tissue samples isolated from adenocarcinoma and squamous cell carcinoma patients when compared to normal tissue. Finally, Bjorkhem-Bergman et al. (Biochem Pharmacol., 2002, 64:159) have described that TRX activity is significantly higher in doxorubicin-resistant, MRP-expressing lung tumour cell lines.

However, all the studies mentioned previously are concerned with the detection of TRX by immunohistochemical means in tumour samples, which limits the usability of the assays described therein for prognostic purposes due to the scarcity of tumour tissue obtained by bronchoscopy in stage IV NSCLC patients and due to the invasive nature of the assay (tumour biopsy). Moreover, all the assays described therein provide a correlation between TRX expression and tumour state but do not teach that said TRX levels could be used as biomarkers for tumour progression in patients, let alone for estimating specifically Time To Progression (TTP).

Accordingly, there is a need in the art for additional NSCLC markers suitable for predicting TTP in a specific manner and present in samples which can be obtained in sufficient amounts and under minimally invasive conditions.

### SUMMARY OF THE INVENTION

The inventors have surprisingly found out that the TRX levels in serum of NSCLC patients that have been treated with chemotherapy correlate with the median TTP of the disease.

Accordingly, in a first aspect, the invention provides a method for predicting the time to progression (TTP) of non-small-cell lung cancer in a patient suffering from said disease and having been treated with chemotherapy, wherein said method comprises
(i) detecting in a sample of said patient the expression levels of thioredoxin,
(ii) comparing the expression levels of thioredoxin in said sample with a reference value,
wherein an increased expression of thioredoxin in comparison with the reference value is indicative of shorter time to progression as a response to said chemotherapy treatment.

In another aspect, the invention provides a method for predicting the time to progression (TTP) of non-small-cell lung cancer in a patient suffering from said disease and having been treated with chemotherapy, wherein said method comprises
(i) detecting in a sample of said patient the expression levels of thioredoxin and the methylation status of the 14-3-3σ gene,
(ii) comparing the expression levels of thioredoxin and the methylation status of the 14-3-3σ gene in said sample with a reference value,
wherein an increased expression of thioredoxin and a decreased level of methylation in the 14-3-3σ gene in comparison with the reference value is indicative of shorter time to progression as a response to said chemotherapy treatment.

In another aspect, the invention provides a kit comprising a first container containing an antibody that is capable of binding specifically to thioredoxin, a second container containing a reagent which modifies unmethylated cytosine and a third container containing primers for amplification of a CpG-containing nucleic acid fragment of the 14-3-3σ gene, wherein the primers distinguish between modified methylated and nonmethylated nucleic acid.

In another aspect, the invention provides the use of a kit as defined above or of a kit comprising a container containing an antibody that is capable of binding specifically to thioredoxin for predicting the time to progression of a patient suffering NSCLC in response to chemotherapy.

### BRIEF DESCRIPTION OF THE DRAWINGS

All figures refer to serum TRX concentrations (in ng/mL) in 107 stage IV NSCLC patients treated with docetaxel/cisplatin.
Figure 1 shows the concentration of TRX in patients classified according to 14-3-3 sigma methylation status, and the correlation between 14-3-3sigma methylation and TRX levels.
Figure 2 shows the concentration of TRX (in ng/mL) in patients classified according to response to doclitaxel/cisplatin, and the correlation between TRX levels and responder vs. non-responder phenotype.
Figure 3 shows the Kaplan-Meier curves representing the time to progression (TTP) of patients classified in quartiles according to serum TRX concentrations, and the correlation between mean time to progression and TRX levels.
Figure 4 shows the Kaplan-Meier curves representing the time to progression (TTP) of patients after grouping together the two middle quartiles. It also shows the correlation between mean time to progression in patients thus classified and TRX expression.

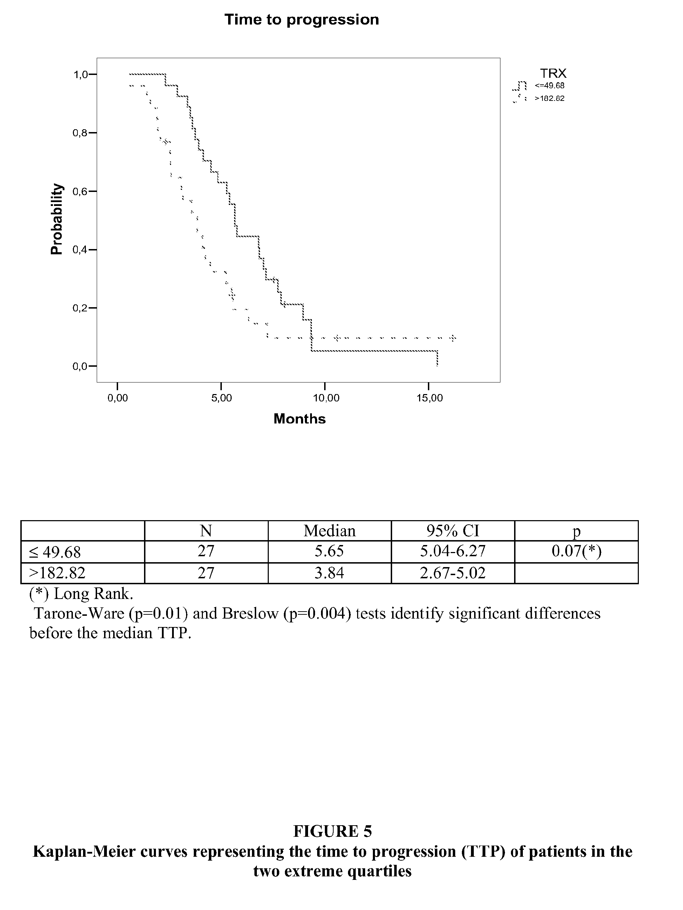

Figure 5 shows the Kaplan-Meier curves representing the time to progression (TTP) of patients in the two extreme quartiles. It also shows the correlation between mean time to progression and TRX expression when comparing these two extreme quartiles.

### DETAILED DESCRIPTION OF THE INVENTION

It is an object of the present invention to provide a method for predicting the TTP of NSCLC patients treated with chemotherapy.

In a first aspect, the invention provides a method for predicting the time to progression (TTP) of non-small-cell lung cancer in a patient suffering from said disease and having been treated with chemotherapy, wherein said method comprises,
(i) detecting in a sample of said patient the expression levels of thioredoxin,
(ii) comparing the expression levels of thioredoxin in said sample with a reference value,
wherein an increased expression of thioredoxin in comparison with the reference value is indicative of shorter time to progression as a response to said chemotherapy treatment.

As used herein, time to progression (TTP) refers to a measure of time after the disease is diagnosed or treated until the disease gets worse. The disease is considered to have progressed if both the diameter and volume of the tumour increased by 25 percent or more of the initial measurements, or if a new lesion is evident on CT or MRI scans.

First, the method of the invention involves detecting the levels of TRX in a sample tissue or body fluid of a patient suffering from NSCLC. The present method can be applied to any type of tissue or body fluid from a patient.

In one embodiment, the method of the invention involves the measurement of TRX levels in tumours or portions thereof which are surgically resected from the patient or obtained by routine biopsy. To simplify conservation and handling of the samples, these can be formalin-fixed and paraffin-embedded.

However, in another aspect of the invention it is preferred that the measurement of TRX levels is carried out in a body fluid from the NSCLC patient. Preferably the body fluid is blood, plasma or serum. More preferably the body fluid is serum. Serum is easily and immediately available from the patient, it suffices to take a blood sample and separate the cells by centrifugation.

The determination of TRX levels in serum can be carried out directly on serum after clotting, centrifugation and elimination of the blood cells.

In a preferred embodiment, the detection of TRX levels is done using antibodies. Any kind of antibody capable of binding TRX specifically can be used in the method of the invention. In particular, the invention contemplates the use of recombinant antibodies, monoclonal antibodies, polyclonal antibodies, intact, or fragments thereof which preserve the capacity to bind to TRX such as, for example, Fab, scFv, etc. fragments, human, humanised or non-human antibodies. The antibodies may be used with or without modification, and may be labelled by covalent or non-covalent attachment of a reporter molecule. A wide variety of reporter molecules is known in the art and may be used.

Any method for detecting the levels of a protein can be used in the present invention. In a still preferred embodiment, the levels of TRX are measured using an immunochemical method. Preferred non limitative examples of such immunochemical methods include enzyme linked immunoabsorbent assay (ELISA), fluorescent immunosorbent assay (FIA), chemical linked immunosorbent assay (CLIA), radioimmunoassay (RIA) and immunoblotting.

The determination of the protein levels of TRX requires the simultaneous measurement of several reference standards. A dose-response curve based on those reference standards is then drawn, and the absolute concentration of human TRX in samples (expressed in ng/mL, nM or any other unit) is calculated from the curve. Other methods known in the art can be used, as long as levels of TRX can be assigned to the samples.

Reference standards are prepared by serial dilution of a calibrator in a buffer. A calibrator is a solution where the absolute concentration of TRX is know. In a preferred embodiment, the calibrator is prepared from pure, lyophilized TRX. Reference standards prepared from the calibrator should comprise the whole range of TRX concentrations in human serum, both in normal and pathological conditions, that go from 0 to 100 ng/mL, and from 0 to 1000 ng/mL, respectively. A standard with no TRX (0 concentration) must always be present.

In a preferred embodiment, the reference value used for determining whether the expression of the TRX protein is "increased" or "decreased" corresponds to the median value of expression levels of TRX measured in a sample obtained by pooling equal amounts of protein from each of the samples obtained from subjects which have not been diagnosed with NSCLC. Once this median value is established, the level of this marker expressed in samples from patients can be compared with this median value, and thus be assigned a level of "increased" or "decreased." In a particular embodiment, an increase in expression above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression. In a particular embodiment, a decrease in expression below the reference value of at least 0.9-fold, 0.75-fold, 0.5-fold, 0.2-fold, 0.1-fold, 0.05-fold, 0.025-fold, 0.02-fold, 0.01-fold, 0.005-fold or even less compared with the reference value is considered as "decreased" expression.

Due to inter-subject variability (e.g. aspects relating to age, race, etc.) it is very difficult (if not practically impossible) to establish absolute reference values for TRX. Thus, in a particular embodiment, the reference values for "increased" or "decreased" TRX expression are determined by calculating percentiles by conventional means involving the testing of a group of samples isolated from normal subjects (i.e. people with no diagnosis of NSCLC) for the expression levels of TRX. The "increased" levels can then be assigned, preferably, to samples wherein expression levels for TRX are equal to or in excels of percentile 50 in the normal population, including, for example, expression levels equal to or in excess to percentile 60 in the normal population, equal to or in excess to percentile 70 in the normal population, equal to or in excess to percentile 80 in the normal population, equal to or in excess to percentile 90 in the normal population, and equal to or in excess to percentile 95 in the normal population.

In a further embodiment, the method of the invention includes, in addition to detecting the levels of TRX, detecting in step (i) the methylation status in the 14-3-3σ gene, wherein a simultaneous increase in thioredoxin expression and a decrease in the methylation status of the 14-3-3σ gene is indicative of shorter time to progression in response to said chemotherapy treatment.

In a preferred embodiment, the methylation status is determined by studying the methylation pattern of the CpG islands in regulatory region of the 14-3-3σ gene. In a more preferred embodiment, the methylation pattern of the CpG is determined in exon 1 of the 14-3-3σ DNA sequence. In a still more preferred embodiment, the state of methylation is determined in a region of the 14-3-3σ sigma gene between CpG dinucleotides 3 and 9, because of the accuracy of the results obtained.

The nucleic acids, preferably DNA, are extracted from the sample by procedures known to the skilled person and commercially available such as the QIAmp Blood Mini kit of QIAGEN. Once the nucleic acid is isolated, the method of the invention includes determining the state of methylation of one or more of those nucleic acids encoding the gene 14-3-3σ and isolated from the subject.

Any method for determining the methylation state of the nucleic acids can be used, such as those described in WO 02/27019, US 6,017,704, US 6,331,393 and US5,786,146, each of which is incorporated herein in its entirety. Determining the methylation state of the nucleic acid includes amplifying the nucleic acid by means of oligonucleotide primers that distinguishes between methylated and unmethylated nucleic acids. One of such methods is described in detail in the examples.

Preferably the method for detecting a methylated CpG-containing nucleic acid includes contacting a nucleic acid-containing specimen with an agent that modifies unmethylated cytosine, amplifying the CpG-containing nucleic acid in the specimen by means of CpG-specific oligonucleotide primers, wherein the oligonucleotide primers distinguish between modified methylated and non-methylated nucleic acid and detecting the methylated nucleic acid. The amplification step is optional and although desirable, is not essential. The method relies on the PCR reaction itself to distinguish between modified (e. g., chemically modified) methylated and unmethylated DNA.

The term "modifies" as used herein means the conversion of an unmethylated cytosine to another nucleotide which will facilitate methods to distinguish the unmethylated from the methylated cytosine. Preferably, the agent modifies unmethylated cytosine to uracil. Preferably, the agent used for modifying unmethylated cytosine is sodium bisulfite, however, other agents that similarly modify unmethylated cytosine, but not methylated cytosine can also be used in the method. Sodium bisulfite (NaHSO₃) reacts readily with the 5,6-double bond of cytosine, but poorly with methylated cytosine. Cytosine reacts with the bisulfite ion to form a sulfonate cytosine reaction intermediate that is susceptible to deamination, giving rise to a sulfonate uracil. The sulfonate group can be removed under alkaline conditions, resulting in the formation of uracil. Uracil is recognized as a thymine by Taq polymerase (C→U→T) and therefore upon PCR, the resultant product contains cytosine only at the position where 5-methylcytosine occurs in the starting template DNA (mC→mC→C).

The methylation state can be determined qualitatively or quantitatively. Well known methods such as fluorescence -based quantitative PCR (using fluorescent primers such as Taqman probes) can be used. Further details can be found in the US patent US 6,331,393.

In a preferred embodiment a qualitative detemination is used, it is quicker and simpler to implement in a lab and the results are accurate. In this embodiment primers able to discriminate between the methylated or unmethylated DNA, as explained before, are used for the PCR, and then the resulting DNA is purified and its methylation status determined for example by separation through agarose gel electrophoresis. A simple visual examination (needs previous staining) under UV light allows classifying the sample as methylated when bands are present in the methylated lane or unmethylated when bands are present in the unmethylated lane only. Synthetically methylated and unmethylated DNA are used as controls.

In a preferred embodiment, the chemotherapy that has been applied to the NSCLC patients prior to predicting the TTP is selected from the group of cisplatin or carboplatin as single agents or a combination selected from cisplatin/paclitaxel, cisplatin/gemcitabine, cisplatin/docetaxel and carboplatin/paclitaxel.

In a preferred embodiment, when the patient has been treated with cisplatin/docetaxel, the TTP is more conveniently estimated by comparing TRX values. In another embodiment, when the patient has been treated with cisplatin/gemcitabine, the TTP is more conveniently estimated by comparing TRX expression values and 14-3-3σ methylation values simultaneously.

In another aspect, the invention provides for kits for the determination of the level of TRX wherein the kits facilitate the employment of methods of the invention. Typically, kits for carrying out a method of the invention contain all the necessary reagents to carry out the method. For example, in one embodiment the kit may comprise a first container containing an antibody or antibodies that are capable of binding specifically to TRX, a second container containing a reagent which modifies unmethylated cytosine and a third container containing primers for amplification of a CpG-containing nucleic acid fragment of the 14-3-3σ gene, wherein the primers distinguish between modified methylated and non-methylated nucleic acid.

In another embodiment, the kit may contain, in addition, a further container containing a anti-human TRX capture antibody. The anti-TRX capture antibody may be immobilized onto a solid surface, such as the well of a microtitre plate or a bead. In this wells or beads coated with this capture antibody, samples to be measured or standards are incubated. After washing, an anti-human TRX detection antibody is added. The anti-TRX detection antibody may be conjugated to a marker such as biotin, alkaline phosphatase or peroxidase. Typically, the kits described above will also comprise one or more other containers, containing for example, wash reagents, and/or other reagents capable of quantitatively detecting the presence of bound antibodies. For example, a signal generator such as a streptavidin peroxidase, may be provided for binding to the detection antibody, and a substrate such as 2,2'- azino-bis-(3-ethylbenzthiazoline-6-sulfonic acid) (ABTS) or tetramethylbenzidine (TMB) may be provided for binding to the signal generator. An acid solution is then added to each well to terminate the signal generation and stabilize the developed colour. The optical density (OD) of each well is then measured and the absolute concentration of human TRX in samples is calculated from the OD thus obtained, using the dose response curve based on reference standards.

Additionally or alternatively, kits of the invention may comprise a competitive ELISA, wherein TRX may be immobilized onto a solid surface. The immobilized TRX may then compete with endogenous TRX present in test sample for binding with an anti-TRX antibody. Additionally or alternatively, the anti-TRX antibody may comprise a marker, for example, biotin, o suitable for binding with a signal generator such as a streptavidin peroxidase.

In accordance with the present invention, the kit contains a second container which contains a reagent which modifies unmethylated cytosine and a second container which contains primers for amplification of a CpG-containing nucleic acid of the 14-3-3σ gene. Preferably, the reagent that modifies unmethylated cytosine is bisulfite.

In the context of the present invention, a compartmentalised kit includes any kit in which reagents are contained in separate containers, and may include small glass containers, plastic containers or strips of plastic or paper. Such containers may allow the efficient transfer of reagents from one compartment to another compartment whilst avoiding cross-contamination of the samples and reagents, and the addition of agents or solutions of each container from one compartment to another in a quantitative fashion. Such kits may also include a container which will accept the test sample, a container which contains the antibody(s) or the oligonucleotides used in the assay, containers which contain wash reagents (such as phosphate buffered saline, Tris-buffers, and like), and containers which contain the detection reagent. Typically, a kit of the present invention will also include instructions for using the kit components to conduct the appropriate methods.

Kits and methods of the invention may be used in conjunction with automated analysis equipment and systems, such as diagnostic systems enabling the analysis of multiple samples and/or multiple biomarkers, for example, the automated bead-based multiplexing BioRad BioPlex 2200 analyser. For example, an automated analyser may be used to determine the level of TRX in conjunction with determining the level of at least one other biomarker in a subject.

In another aspect, the invention provides the use of a kit for predicting the TTP of a patient suffering NSCLC in response to chemotherapy wherein the kit comprises a container containing an antibody that is capable of binding specifically to thioredoxin.

Once the levels of TRX and the 14-3-3-sigma methylation status from a sample are obtained, TTP can be predicted in accordance with the results shown in the examples. Patients with low TRX levels and with 14-3-3-sigma methylation status will have improved TTP if treated with chemotherapy, in particular, docetaxel/cisplatin and cisplatin/gemcitbine.

The invention being thus described, practice of the invention is illustrated by the experimental examples provided below. These examples should not be interpreted as limiting the scope of the claims.

### EXAMPLES

TRX ELISA and 14-3-3σ methylation-specific PCR were performed in baseline serum from 107 stage IV NSCLC patients treated with docetaxel/cisplatin (Table 1).

**Table 1: Characteristics of the patients**

| | | N (%) |
|---|---|---|
| Age median | (range) | 59.8 (32.4-78.9) |
| Sex | Male | 87 (81.3) |
| | Female | 20 (18.7) |
| ECOG | 0 | 27 (25.2) |
| | 1 | 80 (74.8) |
| Stage | IIIB | 19 (17.8) |
| | IV | 88 (82.2) |
| Histology | | |
| | Adenocarcinoma | 46 (43.8) |
| | Squamous | 40 (38.1) |
| | Large cell | 16 (15.2) |
| | Others | 3 (2.9) |
| | NA | 2 |
| Radiotherapy | (yes) | 9 (8.4) |
| Response | | |
| | CR | 1 (1) |
| | PR | 20 (19) |
| | CR+PR | 21 (20) |
| | SD | 50 (46.7) |
| | PD | 34 (31.8) |
| | NE | 2 |
| TRX median | (range) | 97.4 (18.8-763.1) |
| CHFR | | |
| | Methylated | 36 (40.9) |
| | Unmethylated | 52 (59.1) |
| 14-3-3σ | | |
| | Methylated | 43 (48.9) |
| | Unmethylated | 45 (51.1) |
| XRCC3 | | |
| | Met/Met | 18 (16.8) |
| | Thr/Met | 51 (47.7) |
| | Thr/Thr | 38 (35.5) |

### Results:

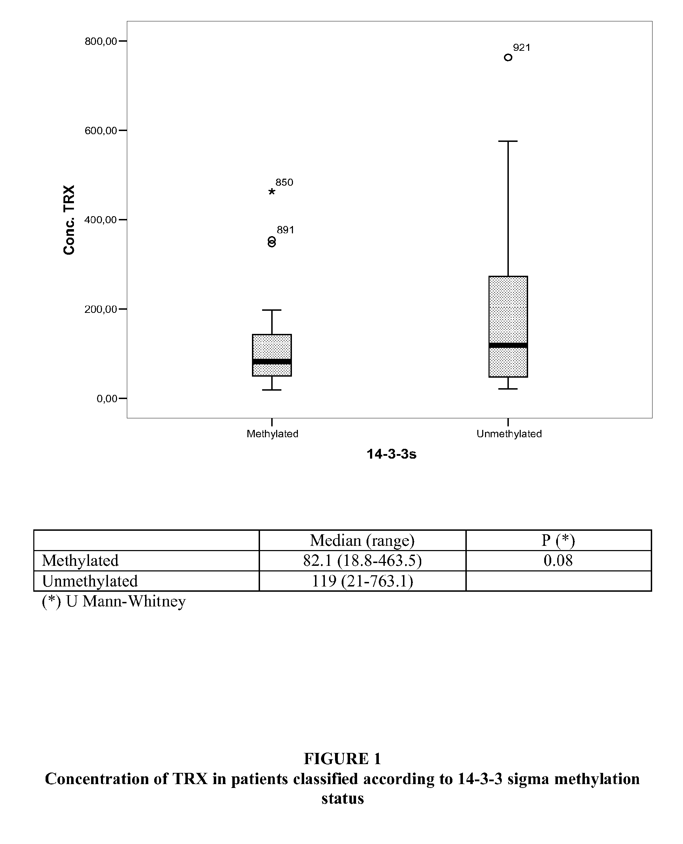

A significant correlation was observed between 14-3-3σ methylation status and TRX levels (Table 2 and figure 1)

**Table 2: Correlation between 14-3-3σ methylation status and TRX levels**

| | ≤ 49.7 | 49.7-182.8 | >182.8 | p |
|---|---|---|---|---|
| Methylated | 11 (25.6%) | 28 (65.1%) | 4 (9.3%) | 0.003 |
| | (47.8%) | (63.6%) | (19%) | |
| Unmethylated | 12 (26.7%) | 16 (35.6%) | 17 (37.8%) | |
| | (52.2%) | (36.4%) | (81%) | |

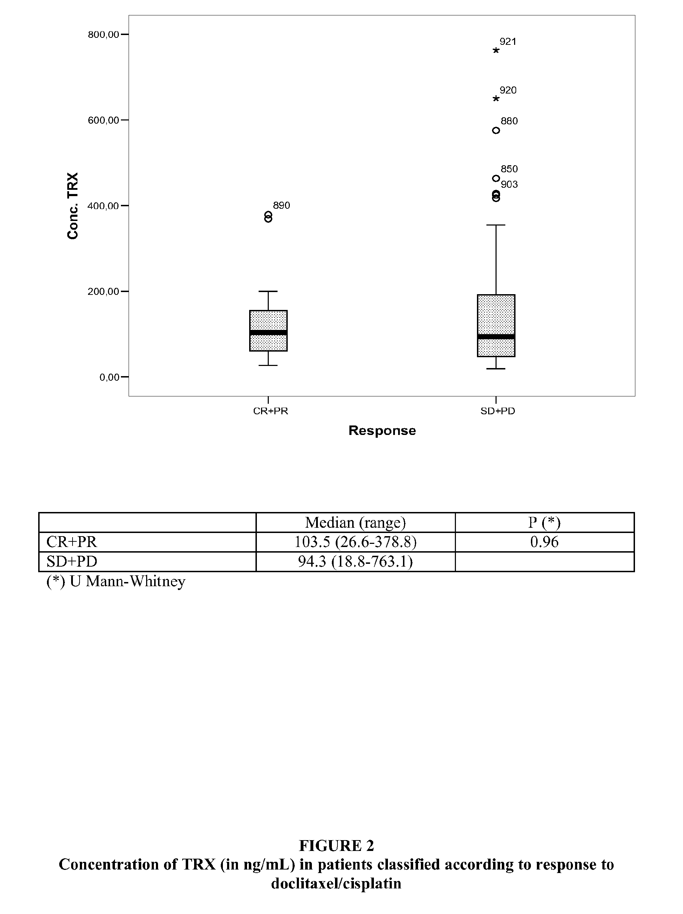

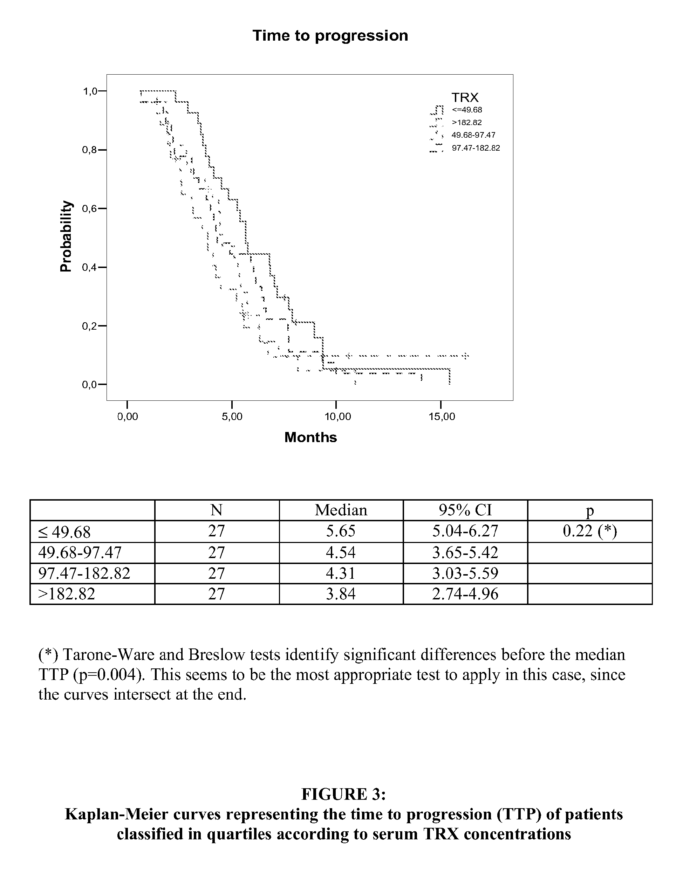

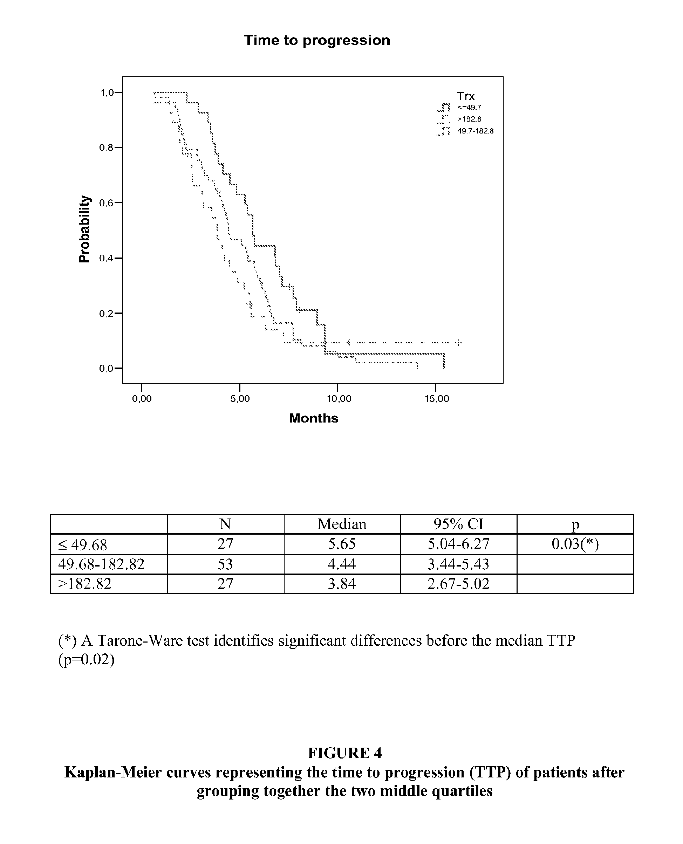

Median TRX levels were 103.5 in responders and 94.3 in non-responders (P=0.96) (Figure 2). No significant correlation was observed between survival rates and TRX levels. However, a clear correlation was observed between time to progression (TTP) and TRX levels. For instance, TTP was 5.6 months (m) for 27 patients with TRX<49.6, 4.4 m for 53 patients with TRX 49.6-182.8, and 3.8 m for 27 patients with TRX >182.8 (P=0.02 in a Tarone-Barlow test) (Figures 3, 4 y 5).

In a Cox multivariate analysis, TRX levels emerged as an independent variable for TTP when 14-3-3σ was included in the model. Hazard ratios: 1.3 for PS1 (P=0.84); 1.05 for 14-3-3σ unmethylated (P=0.22); 1.4 for TRX 49.6-182.8 and 1.95 for TRX >182.8 (P=0.04) (Table 3).

**Table 3: Correlation between TTP and ECOG, 14-3-3σ methylation status and TRX levels.**

| | | HR | 95% CI | p |
|---|---|---|---|---|
| ECOG | 0 | 1 reference | | 0.84 |
| | 1 | 1.34 | 0.63-1.74 | |
| 14-3-3σ | | | | 0.22 |
| | Methylated | 1 reference | | |
| | Unmethylated | 1.05 | 0.81-2.45 | |
| TRX | | | | 0.04 |
| | ≤ 49.7 | 1 reference | | |
| | 49.7-182.8 | 1.41 | 0.81-2.45 | |
| | > 182.8 | 1.95 | 1.01-3.74 | |

**Conclusions:** Serum TRX levels can predict TTP in doc/cis-treated patients. The additional role of 14-3-3σ methylation may be more clearly demonstrated in cis/gemcitabine regimens.

## Claims

1. Method for predicting the time to progression (TTP) of non-small-cell lung cancer in a patient suffering from said disease and having been treated with chemotherapy, wherein said method comprises:
(i) detecting in a sample of said patient the expression levels of thioredoxin,
(ii) comparing the expression levels of thioredoxin in said sample with a reference value,
wherein an increased expression of thioredoxin in comparison with the reference value is indicative of shorter time to progression as a response to said chemotherapy treatment.

2. Method according to claim 1 wherein the sample where the expression level of thioredoxin is detected is serum.

3. Method according to claims 1 or 2 wherein the levels of thioredoxin are detected using a technique selected from the group of enzyme linked immunoabsorbent assay (ELISA), fluorescent immunosorbent assay (FIA), chemical linked immunosorbent assay (CLIA), radioimmuno assay (RIA) and immunoblotting.

4. Method according to claims 1 to 3 further comprising detecting in step (i) the methylation status in the 14-3-3σ gene and wherein a simultaneous increase in thioredoxin expression and a decrease in the methylation status of the 14-3-3σ gene is indicative of shorter time to progression in response to said chemotherapy treatment.

5. The method of claim 4, wherein the state of methylation is determined in a region of the 14-3-3 sigma gene selected from the group of the promoter region, exon 1 or in the region between CpG dinucleotides 3 and 9.

6. The method according to claims 1 to 5 wherein the chemotherapy is selected from cisplatin or carboplatin as single agents or a combination selected from cisplatin/paclitaxel, cisplatin/gemcitabine, cisplatin/docetaxel and carboplatin/paclitaxel.

7. The method according to claim 6 wherein if the chemotherapy administered to the patient is cisplatin/docetaxel, then the prediction of TTP is done by comparing TRX levels and wherein if the chemotherapy is cisplatin/gemcitabine, then the prediction of TTP is done by comparing TRX levels and 14-3-3σ methylation levels.

8. A kit comprising a first container containing an antibody that is capable of binding specifically to thioredoxin, a second container containing a reagent which modifies unmethylated cytosine and a third container containing primers for amplification of a CpG-containing nucleic acid fragment of the 14-3-3σ gene, wherein the primers distinguish between modified methylated and nonmethylated nucleic acid.

9. Use of a kit according to claim 8 or a kit comprising a container containing an antibody that is capable of binding specifically to thioredoxin for predicting the time to progression of a patient suffering NSCLC in response to chemotherapy
